(19) Europäisches Patentamt
European Patent Office
Office européen des brevets

(11) EP 1 537 880 A1

(12) **EUROPEAN PATENT APPLICATION**
published in accordance with Art. 158(3) EPC

(43) Date of publication:
**08.06.2005 Bulletin 2005/23**

(21) Application number: **03795370.0**

(22) Date of filing: **10.09.2003**

(51) Int Cl.[7]: **A61K 45/00**, A61K 31/472, A61K 38/55, A61K 47/30, A61K 47/38, A61K 31/425, A61P 3/10

(86) International application number:
**PCT/JP2003/011570**

(87) International publication number:
**WO 2004/024184 (25.03.2004 Gazette 2004/13)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **11.09.2002 JP 2002266054**

(71) Applicant: **Takeda Pharmaceutical Company Limited**
**Osaka 541-0045 (JP)**

(72) Inventors:
- **AKIYAMA, Yohko**
  **Ohmihachiman-shi, Shiga 523-0898 (JP)**
- **MATSUMOTO, Yukihiro**
  **Kobe-shi, Hyogo 651-0056 (JP)**
- **OI, Satoru**
  **Nara-shi, Nara 631-0033 (JP)**
- **SUZUKI, Nobuhiro**
  **Mino-shi, Osaka 562-0001 (JP)**
- **TSUBOTANI, Shigetoshi**
  **Osaka-shi, Osaka 532-0033 (JP)**

(74) Representative: **Wright, Robert Gordon McRae**
**Elkington and Fife LLP,**
**Prospect House,**
**8 Pembroke Road**
**Sevenoaks, Kent TN13 1XR (GB)**

(54) **SUSTAINED RELEASE PREPARATION**

(57) The sustained-release preparation of the present invention, which contains a dipeptidyl peptidase IV inhibitor and a hydrophilic polymer, can appropriately inhibit the DPP-IV activity, and is superior in convenience or compliance.

EP 1 537 880 A1

## Description

### Technical Field

The present invention relates to a sustained-release preparation comprising a dipeptidyl peptidase IV inhibitor, which is useful for the prophylaxis or treatment of diabetes and the like.

### Background Art

Dipeptidyl peptidase IV (hereinafter sometimes to be abbreviated as DPP-IV) inhibitors are useful as a therapeutic drug for diabetes and the like, since they inhibit inactivation of GLP-1 (glucagon-like peptide-1) in plasma and potentiate its incretin activity (e.g., WO02/062764, WO01/55105, WO02/02560).

As mentioned in detail in the following, however, strong inhibition of DPP-IV activity in living organisms is not necessarily preferable for the living organisms.

For example, it has been reported that a DPP-IV inhibitor potentiates the vasodilating action of substance P, and that DPP-IV activity of nasal mucosa and the density of inflammatory cells in nasal mucosa of patients with chronic rhinosinusitis are in a reverse correlation and DPP-IV activity increased when chronic rhinosinusitis was cured (e.g., The FASEB, vol. 16, p. 1132-1134 (2002)).

Therefore, strong inhibition of DPP-IV activity in diabetic patients with concurrent chronic inflammation is considered to be unpreferable because it causes aggravation of inflammation.

Furthermore, it has been reported that, since GLP-1(9-36) amide, which is a metabolite due to DPP-IV of GLP-1, has a hypoglycemic action, selective DPP-IV activity is important in glucose homeostasis (e.g., Am J Physiol Endocrinol Metab, vol. 282, p. E873-E879 (2002)).

It has been reported, moreover, that the severity of depression and anxiety, which are side effects, correlates with decrease of serum DPP-IV activity in an interferon-$\alpha$ treatment of patients with hepatitis C (e.g., Mol. Psychiatry, vol. 6, p. 475-480 (2001)).

### Disclosure of the Invention

In view of the fact that strong inhibition of DPP-IV activity is not always preferable for living organisms, the present inventors considered that the development of a preparation capable of appropriately inhibiting DPP-IV activity, which is superior in convenience or compliance, is necessary.

The present inventors have studied in search of a preparation capable of appropriately inhibiting DPP-IV activity, which can be administered once a day and, as a result, have found that a sustained-release preparation capable of affording a desired effect can be obtained by combining a DPP-IV inhibitor and a hydrophilic polymer, and further conducted intensive studies, which resulted in the completion of the present invention.

Accordingly, the present invention relates to

1) a sustained-release preparation comprising a dipeptidyl peptidase IV inhibitor and a hydrophilic polymer;
2) the sustained-release preparation of the aforementioned 1), wherein the content of the hydrophilic polymer in the preparation is not less than 5 wt%;
3) the sustained-release preparation of the aforementioned 1), which is used for the prophylaxis or treatment of diabetes;
4) the sustained-release preparation of the aforementioned 1), which is a hypoglycemic agent;
5) a pharmaceutical agent comprising two or more kinds of dipeptidyl peptidase IV inhibitor-containing preparations in combination, which have different release rates of dipeptidyl peptidase IV inhibitor;
6) the pharmaceutical agent of the aforementioned 5), which comprises a sustained-release preparation comprising a dipeptidyl peptidase IV inhibitor and a quick release preparation comprising a dipeptidyl peptidase IV inhibitor;
7) the pharmaceutical agent of the aforementioned 5), which is used for the prophylaxis or treatment of diabetes;
8) the pharmaceutical agent of the aforementioned 5), which is a hypoglycemic agent;
9) a dipeptidyl peptidase IV inhibitor-containing release control preparation capable of decreasing dipeptidyl peptidase IV activity in plasma by 10 to 90% at 1 hr after the administration;
10) a dipeptidyl peptidase IV inhibitor-containing release control preparation capable of decreasing dipeptidyl peptidase IV activity in plasma by 10 to 90% at 8 hr after the administration;
11) a dipeptidyl peptidase IV inhibitor-containing release control preparation capable of decreasing dipeptidyl peptidase IV activity in plasma by 10 to 90% over the period of from 1 hr to 8 hr after the administration;
12) the preparation of any of the aforementioned 9), 10) and 11), which is used for the prophylaxis or treatment of diabetes;

13) the preparation of any of the aforementioned 9), 10) and 11 which is a hypoglycemic agent;
14) a method of treating diabetes, which comprises administering the preparation of any of the aforementioned 9), 10) and 11) to a mammal; and the like.

In the present specification, the DPP-IV inhibitor means a compound that inhibits the enzyme activity of DPP-IV [Classification by International Union of Biochemistry and Molecular Biology (IUBMB):EC3.4.14.5].
This compound may be peptidic or non-peptidic.
In addition, the DPP-IV inhibitor may have different forms before and after administration into living organisms, as long as the DPP-IV inhibitory activity is maintained. In other words, the DPP-IV inhibitor may be an "active metabolite" having a DPP-IV inhibitory activity after becoming a structural variant due to the metabolism in living organisms. Moreover, the DPP-IV inhibitor may be a "prodrug" that changes into an active form due to the reaction of enzyme, gastric acid and the like under physiological conditions in living organisms.
The DPP-IV inhibitory activity can be confirmed by, for example, a method utilizing "the method of Raymond et al. (Diabetes), vol. 47, pp. 1253-1258 (1998)" which is described in the Experimental Example below.
As specific examples of the DPP-IV inhibitor, the following compounds (1)-(8) can be mentioned.
(1) A compound described in WO02/062764, which is represented by the formula:

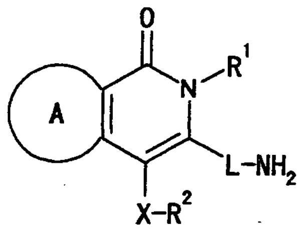 (I)

wherein ring A is an optionally substituted 5- to 10-membered aromatic ring,
$R^1$ and $R^2$ are the same or different and each is an optionally substituted hydrocarbon group or an optionally substituted heterocyclic group,
X is a bond, -O-, -S-, -SO-, $-SO_2-$ or $-NR^3-$ ($R^3$ is a hydrogen atom or an optionally substituted hydrocarbon group); and
L is a divalent hydrocarbon group, or a salt thereof.
As a salt of the compound of the formula (I), a pharamacologically acceptable salt is preferable. Examples of such salt include a salt with an inorganic base, a salt with an organic base, a salt with an inorganic acid, a salt with an organic acid, a salt with a basic or acidic amino acid, and the like.
Preferable examples of the salt with an inorganic base include alkali metal salts such as sodium salt, potassium salt and the like; alkaline earth metal salts such as calcium salt, magnesium salt and the like; aluminum salt; ammonium salt; and the like.
Preferable examples of the salt with an organic base include a salt with trimethylamine, triethylamine, pyridine, picoline, ethanolamine, diethanolamine, triethanolamine, dicyclohexylamine, N,N-dibenzylethylenediamine or the like.
Preferable examples of the salt with an inorganic acid include a salt with hydrochloric acid, hydrobromic acid, nitric acid, sulfuric acid, phosphoric acid or the like.
Preferable examples of the salt with an organic acid include a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid or the like.
Preferable examples of the salt with a basic amino acid include a salt with arginine, lysin, ornithine or the like.
Preferable examples of the salt with an acidic amino acid include a salt with aspartic acid, glutamic acid or the like.
The compound of the formula (I) may be an anhydride or a hydrate, or may be a prodrug.
As preferable examples of a compound represented by the formula (I), the following compounds can be mentioned.

[Compound I-a]

A compound wherein ring A is a benzene ring optionally having 1 or 2 substituent(s) selected from

1) a cyano group;
2) a $C_{1-10}$ alkyl group (preferably ethyl) or a $C_{2-10}$ alkenyl group (preferably ethenyl), each optionally substituted

by a carbamoyl group or a carboxyl group;

3) an optionally substituted hydroxy group [preferably an alkoxy group having 1 to 10 carbon atom(s) (preferably methoxy, isopropoxy) optionally having 1 to 3 substituent(s) selected from a carbamoyl group, a carboxyl group and an alkoxycarbonyl group having 2 to 5 carbon atoms (preferably methoxycarbonyl); a hydroxy group; an aralkyloxy group having 7 to 13 carbon atoms (preferably benzyloxy)] [more preferably carbamoylmethoxy];

4) an acyl group [preferably $C_{1-6}$ alkyl-carbonyl (preferably acetyl), carbamoyl, mono- or di-($C_{1-6}$ alkyl optionally having 1 to 3 substituent(s) selected from halogen atom and $C_{1-6}$ alkoxy-carbonyl)-carbamoyl (preferably methylcarbamoyl, ethylcarbamoyl, propylcarbamoyl, dimethylcarbamoyl, trifluoroethylcarbamoyl, ethoxycarbonylmethylcarbamoyl and the like), $C_{3-10}$ cycloalkyl-carbamoyl (preferably cyclopropylcarbamoyl), $C_{7-13}$ aralkyl-carbamoyl (preferably benzylcarbamoyl), nitrogen-containing heterocycle-carbonyl optionally substituted by hydroxyl (preferably pyrrolidinylcarbonyl, piperidinocarbonyl), $C_{1-6}$ alkylsulfonyl (preferably methylsulfonyl), $C_{1-6}$ alkylsulfinyl (preferably methylsulfinyl), carboxyl, $C_{1-6}$ alkoxy-carbonyl (preferably methoxycarbonyl), thiocarbamoyl];

5) an optionally substituted amino group (preferably carbamoylamino);

6) an optionally substituted thiol group [preferably an alkylthio group having 1 to 10 carbon atom(s) optionally substituted by a carbamoyl group (preferably methylthio)];

7) an optionally substituted heterocyclic group [preferably an aromatic heterocyclic group (preferably furyl, thienyl, oxazolyl, oxadiazolyl, thiazolyl, tetrazolyl, pyridyl, pyrrolyl, triazolyl) or a non-aromatic heterocyclic group (preferably dioxoisoindole, 5-oxooxadiazol-3-yl, 5-oxothiadiazol-3-yl),

each optionally having 1 or 2 substituent(s) selected from a $C_{1-6}$ alkyl group optionally substituted by 1 to 3 halogen atom(s) (preferably methyl, trifluoromethyl), a carboxyl group, an alkoxycarbonyl group having 2 to 8 carbon atoms (preferably ethoxycarbonyl), a cyano group, a carbamoyl group, an amino group, a mono- or di-$C_{2-10}$ alkanoylamino group (e.g., acetylamino, isopentanoylamino), a $C_{1-10}$ alkoxy-carbonylamino group (e.g., methoxycarbonylamino), a carbamoylamino group, a mono- or di-$C_{1-10}$ alkyl-carbamoylamino group (e.g., methylcarbamoylamino, dimethylcarbamoylamino), a $C_{6-14}$ aryl-carbonylamino group (e.g., benzoylamino), a $C_{3-10}$ cycloalkyl-carbonylamino group, a $C_{7-13}$ aralkyloxy-carbonylamino group, a mono- or di-$C_{1-10}$ alkylsulfonylamino group (e.g., methylsulfonylamino, dimethylsulfonylamino), a $C_{6-14}$ arylsulfonylamino group and a $C_{1-6}$ alkoxy-carbamoylamino group (e.g., methoxycarbamoylamino); and

8) an amidino group;

$R^1$ is an alkyl group having 4 to 10 carbon atoms (preferably isobutyl, neopentyl) or a cycloalkyl-alkyl group having 4 to 10 carbon atoms (preferably cyclopropylmethyl);

$R^2$ is an aryl group having 6 to 14 carbon atoms (preferably phenyl) optionally having 1 or 2 substituent(s) selected from halogen atom (preferably fluorine, chlorine) and $C_{1-6}$ alkyl (preferably methyl) ;

X is a bond; and

L is $C_{1-10}$ alkylene (preferably $-CH_2-$).

[Compound I-b]

A compound wherein ring A is a benzene ring optionally having 1 or 2 substituent(s) selected from

1) a $C_{1-10}$ alkyl group (preferably ethyl) or a $C_{2-10}$ alkenyl group (preferably ethenyl), each optionally substituted by a alkoxycarbonyl group having 2 to 8 carbon atoms (preferably ethoxycarbonyl) or a carbamoyl group;

2) an optionally substituted hydroxy group [preferably an alkoxy group having 1 to 10 carbon atom(s) (preferably methoxy) optionally substituted by a carbamoyl group; more preferably carbamoylmethoxy];

3) an acyl group (preferably carbamoyl, thiocarbamoyl, carboxyl);

4) an optionally substituted heterocyclic group [preferably an aromatic heterocyclic group (preferably furyl, thienyl, oxazolyl, oxadiazolyl, thiazolyl, tetrazolyl, pyridyl, pyrrolyl, triazolyl) or a non-aromatic heterocyclic group (preferably 5-oxooxadiazol-3-yl), each optionally having 1 or 2 substituent(s) selected from a $C_{1-6}$ alkyl group (preferably methyl), a carboxyl group, an alkoxycarbonyl group having 2 to 8 carbon atoms (preferably ethoxycarbonyl), a cyano group, a carbamoyl group, an amino group, a mono- or di-$C_{2-10}$ alkanoylamino group (e.g., acetylamino, isopentanoylamino), a $C_{1-10}$ alkoxy-carbonylamino group (e.g., methoxycarbonylamino), a carbamoylamino group, a mono- or di-$C_{1-10}$ alkyl-carbamoylamino group (e.g., methylcarbamoylamino, dimethylcarbamoylamino), a $C_{6-14}$ aryl-carbonylamino group (e.g., benzoylamino), a $C_{3-10}$ cycloalkyl-carbonylamino group, a $C_{7-13}$ aralkyloxy-carbonylamino group, a mono- or di-$C_{1-10}$ alkylsulfonylamino group (e.g., methylsulfonylamino, dimethylsulfonylamino), a $C_{6-14}$ arylsulfonylamino group and a $C_{1-6}$ alkoxy-carbamoylamino group (e.g., methoxycarbamoylamino)];

$R^1$ is an alkyl group having 4 to 10 carbon atoms (preferably isobutyl, neopentyl) or a cycloalkyl-alkyl group having 4 to 10 carbon atoms (preferably cyclopropylmethyl);

$R^2$ is an alkyl group having 1 to 10 carbon atom(s), which is optionally substituted by 1 to 3 halogen atom(s) (preferably butyl) ;

X is -O-; and
L is $C_{1-10}$ alkylene (preferably $-CH_2-$).

Of the compounds represented by the formula (I), 2-[3-(aminomethyl)-4-butoxy-2-isobutyl-1-oxo-1,2-dihydro-6-isoquinolyl]-1,3-thiazole-4-carbonitrile; 2-[3-(aminomethyl)-4-butoxy-2-isobutyl-1-oxo-1,2-dihydro-6-isoquinolyl]-1,3-thiazole-4-carboxylic acid; 2-[3-(aminomethyl)-4-butoxy-2-isobutyl-1-oxo-1,2-dihydro-6-isoquinolyl]-1,3-thiazole-4-carboxamide; ethyl 2-[3-(aminomethyl)-4-butoxy-2-isobutyl-1-oxo-1,2-dihydro-6-isoquinolyl]-1,3-thiazole-4-carboxylate;(E)-3-[3-(aminomethyl)-4-butoxy-2-isobutyl-1-oxo-1,2-dihydro-6-isoquinolyl]-2-propenamide;(E)-3-[3-(aminomethyl)-2-isobutyl-4-phenyl-1-oxo-1,2-dihydro-6-isoquinolyl]-2-propenamide; 3-(aminomethyl)-2-isobutyl-1-oxo-4-phenyl-1,2-dihydro-6-isoquinolinecarboxamide; 2-{[3-(aminomethyl)-2-isobutyl-4-phenyl-1-oxo-1,2-dihydro-6-isoquinolyl]oxy}acetamide; and the like are particularly preferable.

(2) A compound described in WO95/15309 and the like, which is represented by the formula:

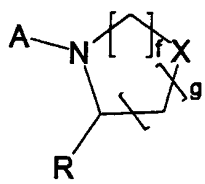

(II)

wherein f is 1 or 2; g is 0, 1 or 2; X is $-CH_2-$, -O-, -S-, -SO-, $-SO_2-$ or $-NR^3-$ ($R^3$ is a hydrogen atom or a $C_{1-6}$ alkyl group) ; R is a hydrogen atom, a cyano group, -CHO, $-B(OH)_2$, $-P(O)(OR^3)$,$-CCR^4$ or $-CH=NR^5$ ($R^4$ is a hydrogen atom, a fluorine atom, a $C_{1-6}$ alkyl group, a cyano group, a nitro group, $-OR^3$, $-CO_2R^3$ or $-COR^3$ ($R^3$ is as defined above); $R^5$ is a phenyl group, a hydroxyl group, $-OR^3$, $-OCOR^3$ or a benzyloxy group ($R^3$ is as defined above)); and A is an optionally substituted amino acid residue, or a salt thereof.

In the formula, as the $C_{1-6}$ alkyl group represented by $R^3$, for example, methyl, ethyl, propyl, isopropyl, butyl, isobutyl, sec.-butyl, t.-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like can be mentioned.

As the amino acid residue of the "optionally substituted amino acid residue" represented by A, a group obtained by removing, from α-amino acid or β-amino acid, OH of the carboxyl group constituting these amino acids can be mentioned.

As used herein, as the α-amino acid, for example, alanine, arginine, asparagine, aspartic acid, glutamine, glutamic acid, glycine, histidine, isoleucine, leucine, lysine, methionine, phenylalanine, proline, serine, threonine, tryptophan, tyrosine, valine, citrulline, ornithine, homocysteine and the like can be mentioned.

As the β-amino acid, for example, β-alanine, β-aminocyclopropanoic acid, β-aminocyclobutanoic acid, β-aminocyclopentanoic acid, β-aminocyclohexanoic acid, β-aminocycloheptanoic acid and β-aminocyclooctanoic acid can be mentioned. The β-amino acid may have an unsaturated bond in the carbon chain constituting the amino acid.

The above-mentioned α-amino acid and β-amino acid may be any of a D form, an L form and a DL form, with preference given to a natural type L form.

The above-mentioned amino acid residue may have 1 or 2 substituent(s) on the amino group or amino acid side chain constituting the amino acid.

As the above-mentioned "substituent on the amino group", an optionally substituted hydrocarbon group, an optionally substituted piperidinyl group and the like are preferable.

As the hydrocarbon group of the "optionally substituted hydrocarbon group", for example, a $C_{1-6}$ alkyl group, a $C_{3-12}$ cycloalkyl group, a $C_{2-6}$ alkenyl group, a $C_{3-12}$ cycloalkenyl group, a $C_{2-6}$ alkynyl group, a $C_{4-12}$ cycloalkadienyl group, a $C_{6-14}$ aryl group (preferably a phenyl group), a $C_{7-15}$ aralkyl group (preferably a benzyl group, a phenethyl group), an adamantyl group, a bicyclo[2.2.1]heptyl group, a bicyclo[3.1.1]heptyl group and the like can be mentioned.

The hydrocarbon group may have 1 to 3 substituent(s) at substitutable position(s), and as such substituents, for example, a halogen atom (preferably fluorine, chlorine); a cyano group; a hydroxyl group optionally substituted by an acyl group; a hydroxymethyl group; a $C_{1-6}$ alkoxy group optionally substituted by 1 to 3 halogen atom(s) (preferably fluorine); and an amino group optionally mono- or di-substituted by an optionally substituted $C_{6-14}$ aryl group or an optionally substituted heterocyclic group can be mentioned.

As used herein, as the acyl group of the "hydroxyl group optionally substituted by an acyl group", for example, the acyl group exemplified as the substituent for ring A in the aforementioned compound I-a can be mentioned.

As the $C_{6-14}$ aryl group of the "optionally substituted $C_{6-14}$ aryl group", for example, a phenyl group, a naphthyl group and the like can be mentioned.

In addition, as the heterocyclic group of the "optionally substituted heterocyclic group", for example, a pyridyl

group, a pyrimidyl group, a pyrazyl group, a quinolyl group, an isoquinolyl group, a quinoxalyl group and the like can be mentioned.

The $C_{6-14}$ aryl group and the heterocyclic group may have 1 to 3 substituents at substitutable position(s). As such substituents, for example, a halogen atom (preferably fluorine, chlorine, bromine); a cyano group; a nitro group; a $C_{1-6}$ alkyl group; a $C_{1-6}$ alkoxy group optionally substituted by 1 to 3 halogen atom(s) (preferably fluorine); a carboxyl group; a carbamoyl group; a $C_{1-6}$ alkylsulfonyl group (preferably a methanesulfonyl group); an aminosulfonyl group optionally mono- or di-substituted by a $C_{1-6}$ alkyl group (preferably a dimethylaminosulfonyl group); and the like can be mentioned.

The substituent of the aforementioned "optionally substituted hydrocarbon group" is particularly preferably a 5-nitro-2-pyridylamino group, a 5-cyano-2-pyridylamino group, a 2-pyrimidylamino group, a 2-pyrazylamino group and the like.

As the substituent of the aforementioned "optionally substituted piperidinyl group", for example, a $C_{1-6}$ alkyl group; a hydroxymethyl group; and the aforementioned "optionally substituted $C_{6-14}$ aryl group" and the "optionally substituted heterocyclic group" exemplified for the "amino group optionally mono- or di-substituted by an optionally substituted $C_{6-14}$ aryl group or an optionally substituted heterocyclic group" can be mentioned. The number of the substituents is, for example, 1 to 3.

As the above-mentioned "substituent on the amino acid side chain", for example, an optionally substituted hydrocarbon group, a hydroxyl group, a $C_{1-10}$ alkoxy group optionally substituted by 1 to 3 halogen atoms (preferably fluorine), an acyl group, an optionally substituted amino group and the like can be mentioned.

As used herein, as the hydrocarbon of the "optionally substituted hydrocarbon group", for example, a $C_{1-10}$ alkyl group, a $C_{3-12}$ cycloalkyl group, a $C_{2-10}$ alkenyl group, a $C_{3-12}$ cycloalkenyl group and the like can be mentioned.

The hydrocarbon group may have 1 to 3 substituent(s) at substitutable position(s). As such substituents, for example, an amino group, a $C_{1-6}$ alkyl-carbonylamino group (preferably an acetylamino group), a hydroxy group, a $C_{1-6}$ alkoxy group, a heterocyclic group (preferably pyridyl) and the like can be mentioned.

As the above-mentioned "acyl group", an optionally substituted nitrogen-containing heterocycle-carbonyl group is preferable. As the "optionally substituted nitrogen-containing heterocycle", for example, a nitrogen-containing heterocycle (preferably pyridine, pyridazine, pyrimidine, pyrazine, imidazole, pyrazole, thiazole, isothiazole, oxazole, isoxazole etc.) optionally having 1 to 3 substituent(s) selected from a halogen atom (preferably fluorine, chlorine, bromine), a cyano group, a nitro group, a $C_{1-6}$ alkyl group (e.g., a trifluoromethyl group) optionally substituted by 1 to 3 halogen atom(s) (preferably fluorine), a $C_{1-6}$ alkoxy group, an amino group optionally mono- or di-substituted by a $C_{1-6}$ alkyl group, a hydroxy group, a carboxyl group and a $C_{1-6}$ alkyl-oxycarbonyl group and the like can be mentioned.

As the substituent of the above-mentioned "optionally substituted amino group", for example, a $C_{1-6}$ alkyl group optionally having 1 to 3 substituent(s) selected from a carboxyl group, a carbamoyl group, a $C_{1-6}$ alkyl-oxycarbonyl group and a nitrogen-containing heterocyclic group (preferably pyridyl) and the like can be mentioned. These substituents may be bonded to a hydroxy group, a carboxyl group, an amino group and the like on the amino acid side chain.

As the salts of the compound represented by the formula (II), those similar to the salts of the compound represented by the formula (I) can be mentioned.

The compound of the formula (II) may be an anhydride or a hydrate, or may be a prodrug.

As preferable examples of the compound represented by the formula (II), N-(N'-substituted glycyl)-2-cyano-pyrrolidine derivatives such as (2S)-1-{{{2-[(5-cyanopyridin-2-yl)amino]ethyl}amino}acetyl}-2-cyano-pyrrolidine (DPP-728) represented by the formula

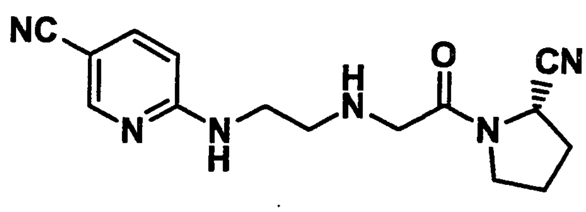

(described in WO98/19998), (2S)-1-{[(3-hydroxy-1-adamantyl)amino]acetyl}-2-cyano-pyrrolidine (LAF237) represented by the formula

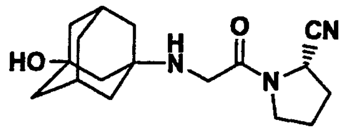

(described in WO00/34241), (2S)-1-{{{2-[(1-pyrimidin-2-ylpiperidin-4-yl)amino]acetyl}-2-cyano-pyrrolidine (described in WO02/30890), (2S)-1-{{{2-[(pyrazin-2-yl)amino]ethyl}amino}acetyl}-2-cyano-pyrrolidine, (S)-1-{1-[5-(N,N-dimethylaminosulfonyl)-2-pyridylamino]-2-methyl-2-propylamino}acetyl-2-pyrrolidinecarbonitrile (K-361) (described in WO02/51836), and the like;
thiazolidine or pyrrolidine derivatives (described in WO01/72290 and the like) such as L-threo-isoleucyl thiazolidine (P32/98) represented by the formula

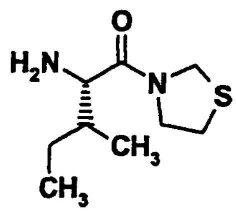

, L-allo-isoleucyl thiazolidine, L-threo-isoleucyl pyrrolidine, L-allo-isoleucyl pyrrolidine, L-valyl pyrrolidine and the like ; and the like can be mentioned.
(3) N-substituted 2-cyanopyrrole and 2-cyanopyrroline derivatives described in WO01/55105. Preferably, (S,S)-1-(2-amino-3,3-dimethylbutyryl)-2,5-dihydro-1H-pyrrole-2-carbonitrile.
(4) Heterocyclic compound described in WO02/02560. Preferably, 7-benzyl-8-[6-(hydroxymethyl)-1,4-diazepan-1-yl]-1,3-dimethyl-3,7-dihydropurine-2,6-dione.
(5) Pyrrolidine derivative fused with cyclopropane, which is described in WO01/68603. Preferably, (1S,3S,5S)-2-[(2S)-2-amino-3,3-dimethylbutyryl]-3-cyano-2-azabicyclo[3.1.0]hexane.
(6) Proline derivative described in WO02/14271. Preferably, (2S)-1-[(2S,4S)-4-(3-chloro-4-cyanophenyl)amino-2-pyrrolidinylcarbonyl]-2-cyanopyrrolidine.
(7) Cyanopyrrolidine derivative described in WO02/38541. Preferably, (2S,4S)-1-[(2S,3S)-2-amino-3-methyl-pentanoyl]-2-cyano-4-fluoropyrrolidine, (2S,4S)-2-cyano-4-fluoro-1-[(1-hydroxymethyl)cyclopentylamino]acetylpyrrolidine, (2S,4S)-2-cyano-4-fluoro-1-(1-hydroxy-3-adamantylamino)acetylpyrrolidine.
(8) Compounds described in WO 02/02560, WO 03/055881, WO 03/040174, WO 03/037327, WO 03/035057, WO 03/035067, WO 03/024942, WO 03/024965, WO 03/004498, WO 03/004496, WO 03/000250, WO 03/002530, WO 03/002531, WO 03/002553, WO 03/000180, WO 03/000181, EP 1258476, WO 0251836, WO 02/68420, US 6432969 and the like; P93/01 and the like.

In the present specification, a "sustained-release preparation" means, for example, a preparation that shows a "drug dissolution rate from a preparation at 30 min after the start of the test" of less than 85% when The Japanese Pharmacopoeia Dissolution Test Method 2 (Paddle Method) is performed using a suitable test solution (900 mL) at a paddle rotation of 100 rpm. As used herein, as the test solution, for example, a test solution that shows a drug concentration of not more than 1/3 of the saturated solubility of the drug, when the drug in a preparation is dissolved by 100% in a test solution, is used. As the test solution, one conventionally employed in the technical field of formulation of preparations, such as water, buffer and the like, is used.

In the present specification, moreover, a preparation that shows a "drug dissolution rate from a preparation at 30 min after the start of the test" of not less than 85%, when The Japanese Pharmacopoeia Dissolution Test Method 2 (Paddle method) is performed under the same conditions as above, is referred to as a quick release preparation.

The present invention relates to "a sustained-release preparation comprising a DPP-IV inhibitor and a hydrophilic polymer". The sustained-release preparation needs not only to contain a DPP-IV inhibitor and a hydrophilic polymer but also satisfy the aforementioned drug dissolution rate.

As used herein, a hydrophilic polymer means a polymer capable of controlling release of a DPP-IV inhibitor by becoming a hydrogel upon absorption of water and diffusing the DPP-IV inhibitor contained in the preparation, or by dissolution of itself in water.

The viscosity of the hydrophilic polymer is, for example, preferably not less than 1 mPa·s, more preferably not less than 4 mPa·s, based on the viscosity of a 2 wt% aqueous solution (measurement temperature: 20°C). In the sustained-release preparation of the present invention, the release period of a DPP-IV inhibitor from the preparation

can be controlled in any way by controlling the viscosity of the hydrophilic polymer used as a base material.

Specific examples of the hydrophilic polymer include hydroxypropyl cellulose (HPC) such as HPC-SSL (trade name, manufactured by Nippon Soda Co., Ltd.) (viscosity of 2 wt% aqueous solution at 20°C: 2.0-2.9 mPa·s), HPC-SL (trade name, manufactured by Nippon Soda Co., Ltd.) (viscosity of 2 wt% aqueous solution at 20°C: 3.0-5.9 mPa·s), HPC-L (trade name, manufactured by Nippon Soda Co., Ltd.) (viscosity of 2 wt% aqueous solution at 20°C: 6.0-10.0 mPa·s), HPC-M (trade name, manufactured by Nippon Soda Co., Ltd.) (viscosity of 2 wt% aqueous solution at 20°C: 150-400 mPa·s), HPC-H (trade name, manufactured by Nippon Soda Co., Ltd.) (viscosity of 2 wt% aqueous solution at 20°C: 1000-4000 mPa·s) and the like;

hydroxypropylmethyl cellulose such as METOLOSE SB-4 (trade name, manufactured by Shin-Etsu Chemical Co., Ltd.) (viscosity of 2 wt% aqueous solution at 20°C: about 4 mPa·s), TC-5RW (trade name, manufactured by Shin-Etsu Chemical Co., Ltd.) (viscosity of 2 wt% aqueous solution at 20°C: about 6 mPa·s), TC-5S (trade name, manufactured by Shin-Etsu Chemical Co., Ltd.) (viscosity of 2 wt% aqueous solution at 20°C: about 15 mPa·s), METOLOSE 60SH-50 (trade name, manufactured by Shin-Etsu Chemical Co., Ltd.) (viscosity of 2 wt% aqueous solution at 20°C: about 50 mPa·s), METOLOSE 65SH-50 (trade name, manufactured by Shin-Etsu Chemical Co., Ltd.) (viscosity of 2 wt% aqueous solution at 20°C: about 50 mPa·s), METOLOSE 90SH-100 (trade name, manufactured by Shin-Etsu Chemical Co., Ltd.) (viscosity of 2 wt% aqueous solution at 20°C: about 100 mPa·s), METOLOSE 90SH-100SR (trade name, manufactured by Shin-Etsu Chemical Co., Ltd.) (viscosity of 2 wt% aqueous solution at 20°C: about 100 mPa·s), METOLOSE 65SH-400 (trade name, manufactured by Shin-Etsu Chemical Co., Ltd.) (viscosity of 2 wt% aqueous solution at 20°C: about 400 mPa·s), METOLOSE 90SH-400 (trade name, manufactured by Shin-Etsu Chemical Co., Ltd.) (viscosity of 2 wt% aqueous solution at 20°C: about 400 mPa·s), METOLOSE 65SH-1500 (trade name, manufactured by Shin-Etsu Chemical Co., Ltd.) (viscosity of 2 wt% aqueous solution at 20°C: about 1500 mPa·s), METOLOSE 60SH-4000 (trade name, manufactured by Shin-Etsu Chemical Co., Ltd.) (viscosity of 2 wt% aqueous solution at 20°C: about 4000 mPa·s), METOLOSE 65SH4000 (trade name, manufactured by Shin-Etsu Chemical Co., Ltd.) (viscosity of 2 wt% aqueous solution at 20°C: about 4000 mPa·s), METOLOSE 90SH-4000 (trade name, manufactured by Shin-Etsu Chemical Co., Ltd.) (viscosity of 2 wt% aqueous solution at 20°C: about 4000 mPa·s), METOLOSE 90SH-4000SR (trade name, manufactured by Shin-Etsu Chemical Co., Ltd.) (viscosity of 2 wt% aqueous solution at 20°C: about 4000 mPa·s), METOLOSE 90SH-30000 (trade name, manufactured by Shin-Etsu Chemical Co., Ltd.) (viscosity of 2 wt% aqueous solution at 20°C: about 30000 mPa·s), METOLOSE 90SH-100000 (trade name, manufactured by Shin-Etsu Chemical Co., Ltd.) (viscosity of 2 wt% aqueous solution at 20°C: about 100000 mPa·s), METOLOSE 90SH-100000SR (trade name, manufactured by Shin-Etsu Chemical Co., Ltd.) (viscosity of 2 wt% aqueous solution at 20°C: about 100000 mPa·s) and the like;

methyl cellulose such as METOLOSE SM15 (trade name, manufactured by Shin-Etsu Chemical Co., Ltd.) (viscosity :about 15 mPa·s, 2 wt% aqueous solution, 20°C), METOLOSE SM25 (trade name, manufactured by Shin-Etsu Chemical Co., Ltd.) (viscosity of 2 wt% aqueous solution at 20°C: about 25 mPa·s), METOLOSE SM100 (trade name, manufactured by Shin-Etsu Chemical Co., Ltd.) (viscosity of 2 wt% aqueous solution at 20°C: about 100 mPa·s), METOLOSE SM400 (trade name, manufactured by Shin-Etsu Chemical Co., Ltd.) (viscosity of 2 wt% aqueous solution at 20°C: about 400 mPa·s), METOLOSE SM1500 (trade name, manufactured by Shin-Etsu Chemical Co., Ltd.) (viscosity of 2 wt% aqueous solution at 20°C: about 1500 mPa·s), METOLOSE SM4000 (trade name, manufactured by Shin-Etsu Chemical Co., Ltd.) (viscosity of 2 wt% aqueous solution at 20°C: about 4000 mPa·s), METOLOSE SM8000 (trade name, manufactured by Shin-Etsu Chemical Co., Ltd.) (viscosity of 2 wt% aqueous solution at 20°C: about 8000 mPa·s) and the like;

polyethylene oxide such as WSR N-12K (trade name, manufactured by Union Carbide Corporation) (viscosity of 2 wt% aqueous solution at 20°C: 400-800 mPa·s), WSR N-60K (trade name, manufactured by Union Carbide Corporation) (viscosity of 2 wt% aqueous solution at 20°C: 2000-4000 mPa·s), WSR 301 (trade name, manufactured by Union Carbide Corporation) (viscosity of 1 wt% aqueous solution at 25°C: 1500-4500 mPa·s), WSR Coagulant (trade name, manufactured by Union Carbide Corporation) (viscosity of 1 wt% aqueous solution at 25°C: 4500-7500 mPa·s), WSR 303 (trade name, manufactured by Union Carbide Corporation) (viscosity of 1 wt% aqueous solution at 25°C: 7500-10000 mPa·s), WSR 308 (trade name, manufactured by Union Carbide Corporation) (viscosity of 1 wt% aqueous solution at 25°C: 10000-15000 mPa·s) and the like;

sodium carboxymethyl cellulose such as Sunrose F-150MC (trade name, manufactured by Nippon Paper Chemicals Co., Ltd.) (viscosity of 1 wt% aqueous solution at 25°C: 1200-1800 mPa·s), Sunrose F-300MC (trade name, manufactured by Nippon Paper Chemicals, Co., Ltd.) (viscosity of 1 wt% aqueous solution at 25°C: 2500-3000 mPa·s), Sunrose F-1000MC (trade name, manufactured by Nippon Paper Chemicals, Co., Ltd.) (viscosity of 1 wt% aqueous solution at 25°C: 8000-12000 mPa·s) and the like; and the like. Two or more kinds of these hydrophilic polymers may be mixed at appropriate ratios for use.

While the content of the DPP-IV inhibitor in a sustained-release preparation varies depending on the kind of DPP-IV inhibitor, the size of the preparation and the like, it is, for example, 1-90 wt%, preferably 5-80 wt%.

While the content of the hydrophilic polymer in a sustained-release preparation varies depending on the content of the DPP-IV inhibitor, the size of the preparation, the kind of a hydrophilic polymer and the like, it is, for example, 5-90 wt%, preferably 10-80 wt%.

As the dosage form of the sustained-release preparation of the present invention, for example, oral agents such as tablet, capsule (including microcapsule), granule, powder and the like; and parenteral agents such as suppository (e.g., rectal suppository, vaginal suppository and the like) and the like can be mentioned, each of which can be safely administered orally or parenterally. Particularly, oral agents such as tablet, capsule, granule and the like are preferable.

The sustained-release preparation of the present invention can be produced by mixing a DPP-IV inhibitor and a hydrophilic polymer and molding the mixture. As used herein, mixing and molding are performed according to conventional methods in the technical field of formulation of preparations. In addition, a pharmacologically acceptable carrier may be used for the above-mentioned mixing and/or molding.

Here, as a pharmacologically acceptable carrier, various organic or inorganic carriers conventionally used as materials for pharmaceutical preparations, such as excipient, lubricant, binder, disintegrant and the like, can be mentioned. Where necessary, additives for pharmaceutical preparation such as preservative, antioxidant, coloring agent, sweetening agent and the like can be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, dextrin, pullulan, light silicic anhydride, synthetic aluminum silicate, magnesium aluminate metasilicate and the like.

Preferable examples of the lubricant include magnesium stearate, calcium stearate, talc, colloidal silica and the like.

Preferable examples of the binder include pregelatinized starch, saccharose, gelatin, gum arabic, methylcellulose, carboxymethylcellulose, sodium carboxymethylcellulose, crystalline cellulose, sucrose, D-mannitol, trehalose, dextrin, pullulan, hydroxypropyl cellulose, hydroxypropylmethylcellulose, polyvinylpyrrolidone and the like.

Preferable examples of the disintegrant include lactose, sucrose, starch, carboxymethylcellulose, calcium carboxymethylcellulose, sodium crosscarmellose, sodium carboxymethyl starch, light silicic anhydride, low-substituted hydroxypropylcellulose and the like.

Preferable examples of the preservative include p-oxybenzoic acid esters, chlorobutanol, benzyl alcohol, phenethyl alcohol, dehydroacetic acid, sorbic acid and the like.

Preferable examples of the antioxidant include sulfite, ascorbate and the like.

Preferable examples of the coloring agent include water-soluble edible tar pigment (e.g., foodcolors such as Food Color Red Nos. 2 and 3, Food Color Yellow Nos. 4 and 5, Food Color Blue Nos. 1 and 2 and the like), water insoluble lake pigment (e.g., aluminum salt of the aforementioned water-soluble edible tar pigment and the like), natural pigments (e.g., beta carotene, chlorophil, red iron oxide, yellow diiron trioxide etc.) and the like.

Preferable examples of the sweetening agent include saccharin sodium, dipotassium glycyrrhizinate, aspartame, stevia and the like.

When the DPP-IV inhibitor to be used for the sustained-release preparation of the present invention is basic, an organic acid may be added to control the dissolution behavior of the sustained-release preparation. Since the solubility of a basic drug is generally greater under the acidic conditions than in the neutral conditions, the drug dissolution property from a sustained-release preparation may vary depending on the environmental pH. In this case, variation in the drug dissolution property due to the environmental pH can be reduced by the use of an organic acid. Suppression of the variation in the drug dissolution property caused by environmental pH is extremely significant in achieving constant efficacy in various patients, because in vivo pH of respective patients may vary.

As the organic acid, for example, citric acid, tartaric acid, ascorbic acid, malic acid, fumaric acid, malonic acid, succinic acid, maleic acid, aspartic acid, glutamic acid and the like can be mentioned. Of these, citric acid, tartaric acid, ascorbic acid and the like are preferable.

While the content of an organic acid in the sustained-release preparation varies depending on the kind and the content of a DPP-IV inhibitor, the size of the preparation and the like, it is, for example, 1-50 wt%, preferably 5-30 wt%.

The sustained-release preparation of the present invention shows low toxicity, causes fewer side effects and can be used as an agent for the prophylaxis or treatment of various diseases to be mentioned later in mammals (e.g., human, bovine, horse, dog, cat, simian, mouse, rat).

The sustained-release preparation of the present invention can be used as an agent for the prophylaxis or treatmet of, for example, diabetes (e.g., type 1 diabetes, type 2 diabetes, gestational diabetes), hyperlipidemia (e.g., hypertriglyceridemia, hypercholesterolemia, hypo-high-density-lipoproteinemia, postprandial hyperlipemia), arteriosclerosis, impaired glucose tolerance (IGT), IFG (Impaired Fasting Glucose), IFG (Impaired Fasting Glycemia), diabetic complications [e.g., neuropathy, nephropathy, retinopathy, cataract, macroangiopathy, osteopenia, hyperosmolar diabetic coma, infectious diseases (e.g., respiratory infection, urinary tract infection, gastrointestinal infection, dermal soft

tissue infections, inferior limb infection and the like), diabetic gangrene, xerostomia, hypacusis, cerebrovascular disorder, peripheral blood circulation disorder and the like], hypoglycemic agent and the like.

Moreover, the sustained-release preparation of the present invention can also prevent progress of impaired glucose tolerance, IFG (Impaired Fasting Glucose) or IFG (Impaired Fasting Glycemia) into diabetes.

Furthermore, the sustained-release preparation of the present invention is also used for pancreatic (β cell) function improvement, pancreatic (β cell) regeneration, promotion of pancreatic (β cell) regeneration and the like.

Since DPP-IV inhibitor is a glucose dependent insulin secretagogue that exhibits selective insulin secreting action in hyperglycemic patients (e.g., patients showing a fasting blood glucose level of not less than 126 mg/dl or the 2-hour value of the 75 g oral glucose tolerance test (75 g OGTT) of not less than 140 mg/dl), the sustained-release preparation of the present invention is useful as a safe agent for the prophylaxis or treatment of diabetes, which is associated with a low risk of vascular complications, hypoglycemic induction and the like, which are undesirable effects of insulin.

While the dose of the sustained-release preparation of the present invention varies depending on the administration subject, administration route, target disease and the like, the DDP-IV inhibitor as an active ingredient is generally given in a single dose of about 0.01-100 mg/kg body weight, preferably 0.05-30 mg/kg body weight, more preferably 0.1-10 mg/kg body weight, in the case of, for example, oral administration to adult diabetic patients. This dose is desirably given 1 or 2 times a day.

In addition, the sustained-release preparation of the present invention is preferably administered during the period that provides a sustained action of DPP-IV inhibitor in living organisms at least from before eating to about 2 hr after eating (preferably 4 hr after eating).

The DPP-IV inhibitor release period of the sustained-release preparation of the present invention in living organisms is preferably 1 to 24 hr, more preferably 2 to 14 hr.

Generally, when a DPP-IV inhibitor is used for the prophylaxis or treatment of diabetes, medication of DPP-IV inhibitor before each meal is necessary because GLP-1, which is a substrate of DPP-IV, is secreted during food intake. However, since the sustained-release preparation of the present invention can release a DPP-IV inhibitor for a long time, a sufficient DPP-IV inhibitory effect can be exhibited even by a single administration per day.

The sustained-release preparation of the present invention can be used in combination with therapeutic agents such as a therapeutic agent for diabetes, a therapeutic agent for diabetic complications, an antihyperlipemia agent, an antihypertensive agent, an antiobestic agent, a diuretic, an antithrombotic agent and the like (hereinafter to be referred to as a combination drug). In this case, the timing of administration of the sustained-release preparation of the present invention and a combination drug is not limited. These may be simultaneously administered to an administration subject or administered in a staggered manner. Moreover, the sustained-release preparation of the present invention and a combination drug may be administered as two kinds of preparations each containing an active ingredient, or may be administered as a single preparation containing both active ingredients.

The dose of the combination drug can be determined as appropriate based on the dose clinically employed. The proportion of the sustained-release preparation of the present invention and a combination drug can be appropriately determined depending on the administration subject, administration route, target disease, condition, combination and the like. When, for example, the administration subject is a human, a combination drug is used in an amount of 0.01-100 parts by weight per 1 part by weight of the DPP-IV inhibitor, which is the active ingredient of the sustained-release preparation of the present invention.

Examples of the above-mentioned therapeutic agent for diabetes include insulin preparations (e.g., animal insulin preparations extracted from pancreas of cattle, swine; human insulin preparations synthesized by genetic engineering techniques using Escherichia coli or yeast; zinc insulin; protamine zinc insulin; fragments or derivatives of insulin (e.g., INS-1 and the like)), insulin sensitizers (e.g., pioglitazone hydrochloride, rosiglitazone (maleate), GI-262570, Reglixane (JTT-501), Netoglitazone (MCC-555), YM-440, KRP-297, CS-011, FK-614, Ragaglitazar (NN-622), Tesaglitazar (AZ-242), BMS-298585, EML-16336, compounds described in WO99/58510 (e.g., (E)-4-[4-(5-methyl-2-phenyl-4-oxazolylmethoxy)benzyloxyimino]-4-phenylbutyric acid)), PPARγ agonists, PPARγ antagonists, PPARγ/α dual agonists, α-glucosidase inhibitors (e.g., voglibose, acarbose, miglitol, emiglitate), biguanides (e.g., phenformin, metformin, buformin and salts thereof such as hydrochloride, fumarate, succinate), insulin secretagogues [sulfonylureas (e.g., tolbutamide, glibenclamide, gliclazide, chlorpropamide, tolazamide, acetohexamide, glyclopyramide, glimepiride, glipizide, glybuzole and the like), repaglinide, senaglinide, nateglinide, mitiglinide or calcium salt hydrate thereof], GLP-1 receptor agonists [e.g., GLP-1, NN-2211, AC-2993 (exendin-4), BIM-51077, Aib(8,35)hGLP-1(7,37) $NH_2$], amyrin agonists (e.g., pramlintide), phosphotyrosine phosphatase inhibitors (e.g., vanadic acid), β3 agonists (e.g., CL-316243, SR-58611-A, UL-TG-307, SB-226552, AJ-9677, BMS-196085, AZ40140), gluconeogenesis inhibitors (e.g., glycogen phosphorylase inhibitors, glucose-6-phosphatase inhibitors, glucagon antagonists, somatostatin receptor agonists), SGLT (sodium-glucose cotransporter) inhibitors (e.g., T-1095) and the like.

Examples of the therapeutic agent for diabetic complications include aldose reductase inhibitors (e.g., Tolrestat, Epalrestat, Zenarestat, Zopolrestat, Minalrestat, Fidarestat, SNK-860, CT-112), neurotrophic factors and increasing drugs thereof (e.g., NGF, NT-3, BDNF, neurotrophin production·secretion promoters described in WO01/14372 (e.g., 4-(4-chlorophenyl)-2-(2-methyl-1-imidazolyl)-5-[3-(2-methylphenoxy)propyl]oxazole) and the like), neuranagenesis stimulators (e.g., Y-128), PKC inhibitors (e.g., LY-333531), AGE inhibitors (e.g., ALT946, pimagedine, pyratoxanthine, N-phenacylthiazolium bromide (ALT766), EXO-226), active oxygen scavengers (e.g., thioctic acid), cerebral vasodilators (e.g., tiapride, mexiletine), and the like.

Examples of the antihyperlipemia agent include statin compounds which are cholesterol synthesis inhibitors (e.g., cerivastatin, pravastatin, simvastatin, lovastatin, atorvastatin, fluvastatin, itavastatin and salts thereof (e.g., sodium salt)), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1, 2, 3, 5-tetrahydro-4,1-benzooxazepin-3-yl]acetyl] piperidine-4-acetic acid and the like), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, plant sterols (e.g., soysterol, γ-oryzanol) and the like.

Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan), calcium antagonists (e.g., manidipine, nifedipine, amlodipine, efonidipine, nicardipine), potassium channel openers (e.g., levcromakalim, L-27152, AL 0671, NIP-121), Clonidine and the like.

Examples of the antiobestic agent include antiobestic agents acting on the central nervous system (e.g., Dexfenfluramine, fenfluramine, phentermine, Sibutramine, amfepramone, dexamphetamine, Mazindol, phenylpropanolamine, clobenzorex), pancreatic lipase inhibitors (e.g., orlistat), β3 agonists (e.g., CL-316243, SR-58611-A, UL-TG-307, SB-226552, AJ-9677, BMS-196085, AZ40140), peptide anorexiants (e.g., leptin, CNTF (Ciliary Neurotropic Factor)), cholecystokinin agonists (e.g., lintitript, FPL-15849) and the like.

Examples of the diuretic include xanthine derivatives (e.g., sodium salicylate and theobromine, calcium salicylate and theobromine), thiazide preparations (e.g., ethiazide, cyclopenthiazide, trichloromethyazide, hydrochlorothiazide, hydroflumethiazide, benzylhydrochlorothiazide, penflutizide, polythiazide, methyclothiazide), antialdosterone agents (e.g., spironolactone, triamterene), carbonate dehydratase inhibitors (e.g., acetazolamide), chlorobenzenesulfonamide agents (e.g., chlortalidone, mefruside, indapamide), azosemide, isosorbide, etacrynic acid, piretanide, bumetanide, furosemide and the like.

Examples of the antithrombotic agent include heparin (e.g., heparin sodium, heparin calcium, dalteparin sodium), warfarin (e.g., warfarin potassium and the like), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

The combination drug is preferably an insulin preparation, an insulin sensitizer, an α-glucosidase inhibitor, a biguanide, an insulin secretagogue (preferably sulfonylurea) or the like.

The present invention further relates to a "pharmaceutical agent comprising two or more kinds of dipeptidyl dipeptidase IV inhibitor-containing preparations in combination, which have different release rates of dipeptidyl dipeptidase IV inhibitor".

Here, the "DPP-IV inhibitor-containing preparation" may be any as long as it contains a DPP-IV inhibitor and may be a sustained-release preparation or a quick release preparation. In addition, a DPP-IV inhibitor release control mechanism of the "DPP-IV inhibitor-containing preparation" is not particularly limited, and may be a preparation wherein a DPP-IV inhibitor is released from the preparation by passive diffusion, a preparation wherein a DPP-IV inhibitor is released along with the degradation of the preparation, a preparation wherein a DPP-IV inhibitor is released in response to the changes in the environmental pH, a preparation wherein a DPP-IV inhibitor is released due to an internal pressure generated by swelling of the inside of the preparation upon absorption of the environmental moisture, or a preparation wherein a DPP-IV inhibitor is immediately released upon degradation or dissolution and the like.

As used herein, "a preparation wherein a DPP-IV inhibitor is released from the preparation by passive diffusion" may be, for example, the aforementioned sustained-release preparation of the present invention [preferably, a matrix tablet using a hydrophilic polymer (e.g., hydroxypropyl cellulose, hydroxypropylmethyl cellulose, polyethylene oxide)], a matrix tablet using a lipophilic base material (e.g., Carnauba wax, hydrogenated castor oil, hydrogenated rapeseed oil, polyglycerine fatty acid ester), a tablet or granule coated with a sustained-release base material (e.g., cellulose polymer such as ethylcellulose and the like; acrylic acid polymer such as aminoalkylmethacrylate copolymer RS [Eudragit RS (trade name, Rohm Pharma Ltd.)], ethyl acrylate-methyl methacrylate copolymer suspension [Eudragit NE (trade name, manufactured by Rohm Pharma Ltd.)] and the like), and the like.

As the "preparation wherein a DPP-IV inhibitor is released along with the degradation of the preparation", for example, a capsule containing poly glycolated glyceride (e.g., Gelucire50/13 (trade name, manufactured by GATTEFOSSE Ltd.) and the like can be mentioned.

As the "preparation wherein a DPP-IV inhibitor is released in response to the changes in the environmental pH", for example, a tablet or granule coated with an enteric base material (e.g., acrylic acid polymers such as methacrylic acid copolymer L [Eudragit L (trade name, Rohm Pharma Ltd.)], methacrylic acid copolymer LD [Eudragit L-30D55

(trade name, Rohm Pharma Ltd.)], methacrylic acid copolymer S [Eudragit S (trade name, manufactured by Rohm Pharma Ltd.)] and the like) and the like can be mentioned.

As the "preparation wherein a DPP-IV inhibitor is released due to an internal pressure generated by swelling of the inside of the preparation upon absorption of the environmental moisture", for example, OROS system (trade name, manufactured by ALZA Corporation) and the like can be mentioned.

As the "preparation wherein a DPP-IV inhibitor is immediately released upon degradation or dissolution", for example, a preparation obtained by mixing a DPP-IV inhibitor and a pharmacologically acceptable carrier and molding the mixture can be mentioned. As used herein, as a pharmacologically acceptable carrier, those similar to the carriers used for the aforementioned sustained-release preparation of the present invention can be mentioned. In addition, the mixing and molding is performed according to conventional methods in the technical field for formulation of preparation.

The release control mechanisms of the "two or more kinds of DPP-IV inhibitor-containing preparations" constituting the pharmaceutical agent of the present invention may be the same or different. The "two or more kinds of DPP-IV inhibitor-containing preparations" may be a single preparation, or plural preparations independent from each other. As used herein, as a single preparation, a single capsule encapsulating two or more kinds of DPP-IV inhibitor-containing preparations; a multilayer tablet (preferably two-layer tablet) having plural release control parts or a nucleated tablet; and the like can be mentioned.

The pharmaceutical agent of the present invention preferably comprises a combination of a DPP-IV inhibitor-containing sustained-release preparation and a DPP-IV inhibitor-containing quick release preparation. By employing such combination, a superior DPP-IV inhibitory action can be obtained for a long time from immediately after administration.

While the content of a DPP-IV inhibitor in a DPP-IV inhibitor-containing preparation varies depending on the kind of the DPP-IV inhibitor, the size of the preparation and the like, it is, for example, 1-90 wt%, preferably 5-80 wt%.

The dosage form of the DPP-IV inhibitor-containing preparation is the same as those mentioned with regard to the aforementioned sustained-release preparation of the present invention.

The pharmaceutical agent of the present invention shows low toxicity, causes fewer side effects and can be used as an agent for the prophylaxis or treatment of various diseases similar to those for the aforementioned sustained-release preparation of the present invention in mammals (e.g., human, cattle, horse, dog, cat, simian, mouse, rat).

The mode of administration of the pharmaceutical agent of the present invention is not particularly limited, and may be any as long as two or more kinds of DPP-IV inhibitor-containing preparations are combined on administration. As such mode of administration, for example, 1) administration of two or more kinds of DPP-IV inhibitor-containing preparations as a single preparation, 2) simultaneous administration of two or more kinds of DPP-IV inhibitor-containing preparations as plural preparations, 3) staggered administration of two or more kinds of DPP-IV inhibitor-containing preparations as plural preparations and the like can be mentioned.

While the dose of the pharmaceutical agent of the present invention varies depending on the administration subject, administration route, target disease and the like, for example, a single dose of a DPP-IV inhibitor, which is the active ingredient, is generally about 0.01-100 mg/kg body weight, preferably 0.05-30 mg/kg body weight, more preferably 0.1-10 mg/kg body weight, for oral administration to an adult diabetic patient. This dose is desirably administered 1 or 2 times a day. In addition, the pharmaceutical preparation of the present invention is preferably administered during the period that provides a sustained action of DPP-IV inhibitor in living organism at least from before eating to about 2 hr after eating (preferably 4 hr after eating).

The pharmaceutical agent of the present invention may be used in combination with similar combination drug as used for the aforementioned sustained-release preparation of the present invention.

The present invention further relates to "a DPP-IV inhibitor-containing release control preparation capable of decreasing the DPP-IV activity in plasma by 10 to 90% (preferably 10 to 85%) at 1 hr after the administration", "a DPP-IV inhibitor-containing release control preparation capable of decreasing the DPP-IV activity in plasma by 10 to 90% (preferably 10 to 85%) at 8 hr after the administration", "a DPP-IV inhibitor-containing release control preparation capable of decreasing the DPP-IV activity in plasma by 10 to 90% (preferably 10 to 85%) at 12 hr after the administration", "a DPP-IV inhibitor-containing release control preparation capable of decreasing the DPP-IV activity in plasma by 10 to 90% (preferably 10 to 85%) over the period of from 1 hr to 8 hr after the administration", "a DPP-IV inhibitor-containing release control preparation capable of decreasing the DPP-IV activity in plasma by 10 to 90% (preferably 10 to 85%) over the period of from 1 hr to 12 hr after the administration" and the like.

As used herein, the plasma of the "DPP-IV activity in plasma" means peripheral intravenous blood plasma. While DPP-IV activity and decrease rate thereof may vary depending on the kind of plasma (e.g., intravenous, arterial or portal vein plasma), any release control preparation capable of decreasing the DPP-IV activity in peripheral intravenous blood plasma by 10 to 90% (preferably 10 to 85%) belong to the "DPP-IV inhibitor-containing release control preparation" of the present invention.

The DPP-IV inhibitor-containing release control preparation of the present invention is capable of decreasing the DPP-IV activity in plasma by 10 to 90%, preferably 10 to 85%, more preferably 10 to 80%, particularly preferably

15 to 75%.

The DPP-IV activity in plasma can be measured by, for example, a method utilizing the "method of Raymond et al., Diabetes, vol. 47, pp. 1253-1258 (1998)" described in the Experimental Example below. The aforementioned decrease rate of the DPP-IV activity in plasma may be different from the above-mentioned values (10, 15, 75, 80, 85, 90%) as long as it is within the general error range. Moreover, depending on the measurement method of the DPP-IV activity in plasma, the decrease rate of the DPP-IV activity in plasma may be different from the above-mentioned values. For example, when, of the measurement conditions of the DPP-IV activity in plasma, the kind of substrate, substrate concentration, reaction time, dilution fold of the plasma and the like are different from the method described in the above-mentioned reference, the decrease rate of the DPP-IV activity in plasma may be greater than the above-mentioned values and, for example, 90% may be a value not less than 95%.

As the DPP-IV inhibitor-containing release control preparation of the present invention, a preparation wherein release of the DPP-IV inhibitor is controlled can be mentioned, from the aforementioned DPP-IV inhibitor-containing preparations of the present invention.

As such preparation, the aforementioned sustained-release preparation of the present invention is preferable. In addition, "a pharmaceutical agent comprising a combination of a DPP-IV inhibitor-containing sustained-release preparation and a DPP-IV inhibitor-containing quick release preparation" and the like, from the aforementioned pharmaceutical agents of the present invention, are also preferable.

The DPP-IV inhibitor-containing release control preparation of the present invention shows low toxicity, causes fewer side effects and can be used as an agent for the prophylaxis or treatment of various diseases similar to those for the aforementioned sustained-release preparation of the present invention in mammals (e.g., human, bovine, horse, dog, cat, simian, mouse, rat).

While the dose of the DPP-IV inhibitor-containing release control preparation of the present invention varies depending on the administration subject, administration route, target disease and the like, for example, a single dose of a DPP-IV inhibitor, which is the active ingredient, is generally about 0.01-100 mg/kg body weight, preferably 0.05-30 mg/kg body weight, more preferably 0.1-10 mg/kg body weight, for oral administration to an adult diabetic patient. This dose is desirably administered 1 or 2 times a day. In addition, the DPP-IV inhibitor-containing release control preparation of the present invention is preferably administered during the period that provides a sustained action of DPP-IV inhibitor in living organism at least from before eating to about 2 hr after eating (preferably 4 hr after eating).

The DPP-IV inhibitor-containing release control preparation of the present invention may be used in combination with similar combination drug as used for the aforementioned sustained-release preparation of the present invention.

The present invention is described in more detail by referring to the following Reference Examples, Examples and Experimental Examples, which are not to be construed as limiting the present invention, and the invention may be modified within the range not deviating from the scope of the present invention.

In the present specification, Compound A means 3-(aminomethyl)-2-isobutyl-1-oxo-4-phenyl-1,2-dihydroisoquinoline-6-carboxamide, Compound B means 2-{[3-(aminomethyl)-2-isobutyl-4-phenyl-1-oxo-1,2-dihydro-6-isoquinolinyl]oxy}acetamide monohydrate, and Compound C means L-threo-isoleucyl thiazolidine (P32/98).

**Reference Example 1**

Compound B (150 mg), lactose (1184 mg), corn starch (360 mg), HPC-L (trade name, manufactured by Nippon Soda Co., Ltd.) (60 mg), carboxymethyl cellulose calcium (trade name: ECG505, manufactured by GOTOKU CHEMICAL COMPANY LTD.) (60 mg), crystalline cellulose (trade name: Avicel, manufactured by Asahi Kasei Chemicals Co., Ltd.) (172 mg) and magnesium stearate (14 mg) were mixed in a mortar. The obtained mixture (200 mg) was compressed using a hydraulic pump press (manufactured by RIKEN SEIKI) to give a tablet having a diameter of 8 mm.

**Reference Example 2**

Production of Compound A

Compound A used in the following Examples 1-6, 8-10, 16 and 17 was produced as follows.

To a solution of Compound A hydrochloride (2.04 g, 5 mmol) in water (20 mL) was added 1N sodium hydroxide (10 mL) and the obtained mixture was stirred at room temperature for 10 min. The reaction mixture was poured into water and extracted with ethyl acetate. The extract was washed with brine, dried over anhydrous magnesium sulfate and concentrated under reduced pressure. The obtained crystals were recrystallized from ethyl acetate to give Compound A (0.87 g, 82.9%) as crystals.

| data of powder X-ray crystal diffraction | |
|---|---|
| diffraction angle: 2θ (°) | spacing: d value (angstrom) |
| 5.98<br>7.88<br>8.44<br>17.1 | 14.8<br>11.2<br>10.5<br>5.19 |

**Reference Example 3**

Production of Compound A

Compound A used in the following Examples 7 and 18-23 was produced as follows.

1) To a mixture of 3-(aminomethyl)-2-isobutyl-1-oxo-4-phenyl-1,2-dihydroisoquinoline-6-carbonitrile (0.80 g, 2.4 mmol), 1N aqueous sodium hydroxide solution (0.48 mL, 0.48 mmol) and water (1.42 mL) was added dimethyl sulfoxide (2.0 ml) and the mixture was stirred at 85°c for 1 hr. After cooling to room temperature over 1 hr, the reaction mixture was stirred for 1 hr in an ice bath. The precipitated crystals were collected by filtration, washed with water (1 mL) and dried at 50°c under the reduced pressure to give Compound A 1/2 dimethyl sulfoxide (0.88 g, 93%) as colorless crystals.
2) A mixture of Compound A 1/2 dimethyl sulfoxide (10.0 g, 26 mmol) and methanol (40 mL) was stirred at 60°c. To the obtained solution was added 1N hydrochloric acid (27 mL) at 60°c to adjust the solution pH to 1.5, and then activated carbon (0.5 g) was added. The obtained mixture was stirred at 60°c for 10 min. The activated carbon was filtrated and washed with methanol-water (2:1, 10 mL). The filtrate and the washing solution were combined, and 5% aqueous ammonia (15 mL) was added under stirring at 60°c to adjust the solution pH to 7.3. After water (1.3 mL) was added dropwise to the reaction mixture the reaction mixture was cooled to room temperature over 1 hr and stirred for 1 hr in an ice bath. The precipitated crystals were collected by filtration, washed with cooled methanol-water (1:1, 15 mL) and dried at 50°c under the reduced pressure. A mixture of the obtained crystals (10 g) and ethyl acetate (100 mL) was stirred at 75°c for 1 hr. The obtained suspension was cooled to room temperature over 1 hr, and stirred for 1 hr in an ice bath. The crystals were collected by filtration, washed with cooled ethyl acetate (20 mL) and dried at 50°c under the reduced pressure to give Compound A (7.5 g, 85%) as colorless crystals.

| data of powder X-ray crystal diffraction | |
|---|---|
| diffraction angle: 2θ (°) | spacing: d value (angstrom) |
| 8.98<br>10.0<br>16.0<br>17.1<br>22.9<br>24.8<br>25.8 | 9.84<br>8.82<br>5.55<br>5.19<br>3.88<br>3.59<br>3.46 |

**Reference Example 4**

production of Compound B

Compound B used in the following Examples 13-15 was produced as follows.

2-{[3-(Aminomethyl)-2-isobutyl-4-phenyl-1-oxo-1,2-dihydro-6-isoquinolinyl]oxy}acetamide (1.0 g, 2.6 mmol) was dissolved in ethanol (10 mL) under heating, and water (10 mL) was added to the solution. Then, seed crystals of Compound B were added, and the solution was allowed to cool to room temperature. The precipitated crystals were collected by filtration, washed with water to give Compound B (0.31 g, 31.0%) as crystals.

elemental analysis as $C_{22}H_{25}N_3O_3\ H_2O$
Calculated : C, 66.48; H, 6.85; N, 10.57.
Found : C, 66.51; H, 7.05; N, 10.50.

| data of powder X-ray crystal diffraction | |
|---|---|
| diffraction angle: 2θ (°) | spacing: d value (angstrom) |
| 7.50 | 11.78 |
| 11.2 | 7.89 |
| 13.7 | 6.46 |
| 14.7 | 6.04 |
| 18.5 | 4.80 |
| 19.8 | 4.47 |
| 20.4 | 4.35 |
| 27.4 | 3.25 |

**Example 1**

Compound A (100 mg), HPC-L (trade name, manufactured by Nippon Soda Co., Ltd.) (180 mg) and HPC-M (trade name, manufactured by Nippon Soda Co., Ltd.) (720 mg) were mixed in a mortar. The obtained mixture (200 mg) was compressed using a hydraulic pump press (manufactured by RIKEN SEIKI) to give a tablet having a diameter of 8 mm.

**Example 2**

Compound A (100 mg), HPC-L (trade name, manufactured by Nippon Soda Co., Ltd.) (450 mg) and HPC-M (trade name, manufactured by Nippon Soda Co., Ltd.) (450 mg) were mixed in a mortar. The obtained mixture (200 mg) was compressed using a hydraulic pump press (manufactured by RIKEN SEIKI) to give a tablet having a diameter of 8 mm.

**Example 3**

Compound A (100 mg), HPC-L (trade name, manufactured by Nippon Soda Co., Ltd.) (720 mg) and HPC-M (trade name, manufactured by Nippon Soda Co., Ltd.) (180 mg) were mixed in a mortar. The obtained mixture (200 mg) was compressed using a hydraulic pump press (manufactured by RIKEN SEIKI) to give a tablet having a diameter of 8 mm.

**Example 4**

Compound A (100 mg), Polyox WSR303 (trade name, manufactured by Union Carbide Corporation) (720 mg) and polyethylene glycol 6000 (180 mg) were mixed in a mortar. The obtained mixture (200 mg) was compressed using a hydraulic pump press (manufactured by RIKEN SEIKI) to give a tablet having a diameter of 8 mm.

**Example 5**

Compound A (100 mg), Polyox WSR303 (trade name, manufactured by Union Carbide Corporation) (450 mg) and polyethylene glycol 6000 (450 mg) were mixed in a mortar. The obtained mixture (200 mg) was compressed using a hydraulic pump press (manufactured by RIKEN SEIKI) to give a tablet having a diameter of 8 mm.

**Example 6**

Compound A (100 mg), Polyox WSR303 (trade name, manufactured by Union Carbide Corporation) (180 mg) and polyethylene glycol 6000 (720 mg) were mixed in a mortar. The obtained mixture (200 mg) was compressed using a hydraulic pump press (manufactured by RIKEN SEIKI) to give a tablet having a diameter of 8 mm.

**Example 7**

Compound A (300 mg) and HPC-M (trade name, manufactured by Nippon Soda Co., Ltd.) (700 mg) were mixed in a mortar. The obtained mixture (200 mg) was compressed using a hydraulic pump press (manufactured by RIKEN SEIKI) to give a tablet having a diameter of 8 mm.

**Example 8**

Compound A (78 mg), TC-5S (trade name, manufactured by Shin-Etsu Chemical Co., Ltd.) (47 mg), METOLOSE 90SH4000 (trade name, manufactured by Shin-Etsu Chemical Co., Ltd.) (190 mg) and magnesium stearate (2.4 mg) were mixed in a mortar. The obtained mixture (243 mg) was compressed using a hydraulic pump press (manufactured by RIKEN SEIKI) to give a tablet having a diameter of 8 mm.

**Example 9**

Compound A (79 mg), TC-5S (trade name, manufactured by Shin-Etsu Chemical Co., Ltd.) (119 mg), METOLOSE 90SH4000 (trade name, manufactured by Shin-Etsu Chemical Co., Ltd.) (118 mg) and magnesium stearate (2.4 mg) were mixed in a mortar. The obtained mixture (243 mg) was compressed using a hydraulic pump press (manufactured by RIKEN SEIKI) to give a tablet having a diameter of 8 mm.

**Example 10**

Compound A (79 mg), TC-5S (trade name, manufactured by Shin-Etsu Chemical Co., Ltd.) (190 mg), METOLOSE 90SH4000 (trade name, manufactured by Shin-Etsu Chemical Co., Ltd.)(47 mg) and magnesium stearate (2.4 mg) were mixed in a mortar. The obtained mixture (243 mg) was compressed using a hydraulic pump press (manufactured by RIKEN SEIKI) to give a tablet having a diameter of 8 mm.

**Example 11**

Compound C (100 mg), HPC-L (trade name, manufactured by Nippon Soda Co., Ltd.) (450 mg) and HPC-M (trade name, manufactured by Nippon Soda Co., Ltd.) (450 mg) were mixed in a mortar. The obtained mixture (200 mg) was compressed using a hydraulic pump press (manufactured by RIKEN SEIKI) to give a tablet having a diameter of 8 mm.

**Example 12**

Compound C (100 mg), HPC-L (trade name, manufactured by Nippon Soda Co., Ltd.) (720 mg) and HPC-M (trade name, manufactured by Nippon Soda Co., Ltd.) (180 mg) were mixed in a mortar. The obtained mixture (200 mg) was compressed using a hydraulic pump press (manufactured by RIKEN SEIKI) to give a tablet having a diameter of 8 mm.

**Example 13**

Compound B (75 mg) and HPC-L (trade name, manufactured by Nippon Soda Co., Ltd.) (925 mg) were mixed in a mortar. The obtained mixture (200 mg) was compressed using a hydraulic pump press (manufactured by RIKEN SEIKI) to give a tablet having a diameter of 8 mm.

**Example 14**

Compound B (37.5 mg), HPC-L (trade name, manufactured by Nippon Soda Co., Ltd.) (370 mg) and HPC-M (trade name, manufactured by Nippon Soda Co., Ltd.) (92.5 mg) were mixed in a mortar. The obtained mixture (200 mg) was compressed using a hydraulic pump press (manufactured by RIKEN SEIKI) to give a tablet having a diameter of 8 mm.

**Example 15**

Compound B (37.5 mg), HPC-L (trade name, manufactured by Nippon Soda Co., Ltd.) (231.3 mg) and HPC-M (trade name, manufactured by Nippon Soda Co., Ltd.) (231.3 mg) were mixed in a mortar. The obtained mixture (200

mg) was compressed using a hydraulic pump press (manufactured by RIKEN SEIKI) to give a tablet having a diameter of 8 mm.

**Example 16**

Compound A (120 mg) was added to Gelucire 50/13 (trade name, manufactured by Gattefosse) (2280 mg) melt at about 70°c, and the mixture was stirred. The obtained mixture (400 mg) was filled in a gelatin capsule (No. 1) and left at room temperature for 24 hr to solidify.

**Example 17**

Gelucire 50/13 (trade name, manufactured by Gattefosse) (2052 mg) was added to Carnauba wax (trade name: polishing wax 103, manufactured by Freund Corporation) (228 mg) melt at about 90°c, and the mixture was kept at about 80°c. Compound A (120 mg) was added to the obtained mixture and the mixture was stirred. The obtained mixture (400 mg) was filled in a gelatin capsule (No. 1) and left at room temperature for 24 hr to solidify.

**Example 18**

Compound A (100 mg), hydrogenated castor oil (trade name: Lubri wax 101, manufactured by Freund Corporation) (500 mg), citric acid (100 mg) and lactose (300 mg) were mixed in a mortar. The obtained mixture (200 mg) was compressed using a hydraulic pump press (manufactured by RIKEN SEIKI) to give a tablet having a diameter of 8 mm.

**Example 19**

Compound A (100 mg), hydrogenated castor oil (trade name: Lubri wax 101, manufactured by Freund Corporation) (500 mg), citric acid (100 mg) and lactose (300 mg) were mixed in a mortar. The obtained mixture (200 mg) was compressed using a hydraulic pump press (manufactured by RIKEN SEIKI) to give a tablet having a diameter of 8 mm.

**Example 20**

Compound A (100 mg), HPC-L (trade name, manufactured by Nippon Soda Co., Ltd.) (500 mg), HPC-H (trade name, manufactured by Nippon Soda Co., Ltd.) (300 mg) and citric acid (100 mg) were mixed in a mortar. The obtained mixture (200 mg) was compressed using a hydraulic pump press (manufactured by RIKEN SEIKI) to give a tablet having a diameter of 8 mm.

**Example 21**

Compound A (300 mg), HPC-M (trade name, manufactured by Nippon Soda Co., Ltd.) (400 mg) and citric acid (300 mg) were mixed in a mortar. The obtained mixture (200 mg) was compressed using a hydraulic pump press (manufactured by RIKEN SEIKI) to give a tablet having a diameter of 8 mm.

**Example 22**

Compound A (300 mg), METOLOSE 90SH-4000 (trade name, manufactured by Shin-Etsu Chemical Co., Ltd.) (500 mg) and citric acid (200 mg) were mixed in a mortar. The obtained mixture (200 mg) was compressed using a hydraulic pump press (manufactured by RIKEN SEIKI) to give a tablet having a diameter of 8 mm.

**Example 23**

Compound A (300 mg), METOLOSE 90SH-4000 (trade name, manufactured by Shin-Etsu Chemical Co., Ltd.) (250 mg), HPC-M (trade name, manufactured by Nippon Soda Co., Ltd.) (250 mg) and citric acid (200 mg) were mixed in a mortar. The obtained mixture (200 mg) was compressed using a hydraulic pump press (manufactured by RIKEN SEIKI) to give a tablet having a diameter of 8 mm.

### Example 24

Compound B (600 mg), METOLOSE 65SH-4000 (trade name, manufactured by Shin-Etsu Chemical Co., Ltd.) (400 mg), TC5-RW (trade name, manufactured by Shin-Etsu Chemical Co., Ltd.) (800 mg), mannitol (180 mg) and magnesium stearate (20 mg) were mixed in a mortar. The obtained mixture (400 mg) was compressed using a hydraulic pump press (manufactured by RIKEN SEIKI) to give a tablet having a diameter of 11 mm.

### Example 25

Compound B (900 mg), METOLOSE 65SH-400 (trade name, manufactured by Shin-Etsu Chemical Co., Ltd.) (600 mg), TC5-RW (trade name, manufactured by Shin-Etsu Chemical Co., Ltd.) (600 mg), mannitol (840 mg), yellow diiron trioxide (30 mg) and magnesium stearate (30 mg) were mixed in a mortar. The obtained mixture (400 mg) was compressed using a hydraulic pump press (manufactured by RIKEN SEIKI) to give a tablet having a diameter of 11 mm.

### Example 26

Compound B (450 mg), HPC-L (trade name, manufactured by Nippon Soda Co., Ltd.) (1020 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

### Example 27

Compound B (450 mg), Polyox WSR303 (trade name, manufactured by Union Carbide Corporation) (150 mg), TC5-RW (trade name, manufactured by Shin-Etsu Chemical Co., Ltd.) (450 mg), mannitol (420 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

### Example 28

Compound B (450 mg), METOLOSE 60SH-4000 (trade name, manufactured by Shin-Etsu Chemical Co., Ltd.) (150 mg), TC5-RW (trade name, manufactured by Shin-Etsu Chemical Co., Ltd.) (450 mg), mannitol (420 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

### Example 29

Compound B (450 mg), METOLOSE 90SH-4000 (trade name, manufactured by Shin-Etsu Chemical Co., Ltd.) (225 mg), TC5-RW (trade name, manufactured by Shin-Etsu Chemical Co., Ltd.) (375 mg), mannitol (420 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

### Example 30

Compound B (900 mg), METOLOSE 60SH-50 (trade name, manufactured by Shin-Etsu Chemical Co., Ltd.) (1200 mg), mannitol (840 mg), yellow diiron trioxide (30 mg) and magnesium stearate (30 mg) were mixed in a mortar. The obtained mixture (400 mg) was compressed using a hydraulic pump press (manufactured by RIKEN SEIKI) to give a tablet having a diameter of 11 mm.

### Example 31

Compound B (450 mg), METOLOSE 90SH-400 (trade name, manufactured by Shin-Etsu Chemical Co., Ltd.) (300 mg), TC5-RW (trade name, manufactured by Shin-Etsu Chemical Co., Ltd.) (300 mg), mannitol (420 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

**Example 32**

Compound B (450 mg), METOLOSE 65SH-50 (trade name, manufactured by Shin-Etsu Chemical Co., Ltd.) (600 mg), mannitol (420 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

**Example 33**

Compound B (450 mg), METOLOSE 90SH-100 (trade name, manufactured by Shin-Etsu Chemical Co., Ltd.) (600 mg), mannitol (420 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

**Example 34**

Compound B (450 mg), METOLOSE 65SH-1500 (trade name, manufactured by Shin-Etsu Chemical Co., Ltd.) (225 mg), TC5-RW (trade name, manufactured by Shin-Etsu Chemical Co., Ltd.) (375 mg), mannitol (420 mg), yellow diiron trioxide (15 mg) and magnesium stearate (15 mg) were mixed in a mortar. The obtained mixture (400 mg) was compressed using a hydraulic pump press (manufactured by RIKEN SEIKI) to give a tablet having a diameter of 11 mm.

**Example 35**

Compound B (900 mg), METOLOSE 90SH-400 (trade name, manufactured by Shin-Etsu Chemical Co., Ltd.) (450 mg), METOLOSE 60SH-50 (trade name, manufactured by Shin-Etsu Chemical Co., Ltd.) (1200 mg), mannitol (390 mg), yellow diiron trioxide (30 mg) and magnesium stearate (30 mg) were mixed in a mortar. The obtained mixture (400 mg) was compressed using a hydraulic pump press (manufactured by RIKEN SEIKI) to give a tablet having a diameter of 11 mm.

**Example 36**

Compound B (4190 mg), METOLOSE 90SH-30000 (trade name, manufactured by Shin-Etsu Chemical Co., Ltd.) (8000 mg), METOLOSE SB-4 (trade name, manufactured by Shin-Etsu Chemical Co., Ltd.) (2000 mg), crystalline cellulose (2000 mg), mannitol (3570 mg), yellow diiron trioxide (40 mg) and magnesium stearate (200 mg) were mixed in a mortar. The obtained mixture (500 mg) was compressed using a hydraulic pump press (manufactured by RIKEN SEIKI) to give a tablet having a diameter of 11 mm.

**Example 37**

Compound B (2095 mg), METOLOSE 90SH-100000 (trade name, manufactured by Shin-Etsu Chemical Co., Ltd.) (2000 mg), crystalline cellulose (2000 mg), mannitol (3785 mg), yellow diiron trioxide (20 mg) and magnesium stearate (100 mg) were mixed in a mortar. The obtained mixture (500 mg) was compressed using a hydraulic pump press (manufactured by RIKEN SEIKI) to give a tablet having a diameter of 11 mm.

**Example 38**

A tablet (333 mg) containing Compound B (104.7 mg), METOLOSE 90SH-4000 (trade name, manufactured by Shin-Etsu Chemical Co., Ltd.) (116.6 mg), crystalline cellulose (33.3 mg), mannitol (71.8 mg) and magnesium stearate (6.6 mg) was prepared by a conventional method.

**Experimental Example 1**

The sustained-release preparation of the present invention was evaluated by a dissolution test.
To be specific, the tablets obtained in Examples 1-3 were subjected to a dissolution test (Paddle Method, 0.1% cetyltrimethylammoniumbromide (CTAB)-containing The Japanese Pharmacopoeia Degradation Test2nd fluid (hereinafter to be abbreviated as CTAB-containing The Japanese Pharmacopoeia Degradation Test 2nd fluid) 500 mL, number of rotation 100 rpm). The results are shown in Table 1. In addition, as a control group, the powder of compound

A was subjected to a similar dissolution test and found to show a dissolution rate of 99% after 10 min.

Table 1

| Dissolution rate (%) | | | |
|---|---|---|---|
| Time (h) | Example 1 | Example 2 | Example 3 |
| 0 | 0 | 0 | 0 |
| 1 | 7 | 10 | 19 |
| 2 | 11 | 17 | 32 |
| 4 | 20 | 31 | 56 |
| 6 | 30 | 43 | 79 |
| 8 | 40 | 57 | 93 |
| 10 | 49 | 69 | 99 |
| 12 | 57 | 76 | 99 |
| 14 | 65 | 84 | 98 |
| 16 | 72 | 90 | 98 |
| 18 | 79 | 93 | 98 |

From the above, the sustained-release preparation of the present invention is confirmed to have a superior sustained release action. In addition, it became clear that the sustained-release of the preparation can be controlled by changing the mixing ratio of the hydrophilic polymer contained in the sustained-release preparation of the present invention.

**Experimental Example 2**

The sustained-release preparation of the present invention was evaluated by a dissolution test.
To be specific, a dissolution test of the tablet obtained in Example 13 was performed in the same manner as in Experimental Example 1. The results are shown in Table 2. In addition, as a control group, the tablet of Reference Example 1 was subjected to a similar dissolution test and found to show a dissolution rate of 100% after 10 min.

Table 2

| Time (h) | Dissolution rate (%) |
|---|---|
| 0 | 0 |
| 1 | 8 |
| 2 | 24 |
| 4 | 53 |
| 6 | 85 |
| 8 | 95 |
| 10 | 99 |

From the above, the sustained-release preparation of the present invention is confirmed to have a superior sustained release action.

**Experimental Example 3**

The plasma dipeptidyl peptidase IV (DPP-IV)-inhibitory activity of the pharmaceutical agent of the present invention was evaluated using Beagles.
To be specific, one tablet obtained in Example 13, or one tablet obtained in Example 13 and one tablet obtained in Reference Example 1, was/were orally administered to a Beagle fasted overnight. Blood was drawn from the vein

of the forepaw before administration and 0.5, 1, 2, 4, 8 hr after administration, and the DPP-IV activity in the obtained heparin plasma was measured as in the following.

The reaction was carried out according to the method of Raymond et al. (Diabetes, vol. 47, pp. 1253-1258, 1998) using a 96 well flat-bottomed plate at 30°C. A 1mM aqueous glycyl·prolyl-p-nitroanilide (Gly-Pro-p-NA; manufactured by Bachem AG) solution (100 µL) was added to a mixture of water (70 µl) and 1M Tris-hydrochloride buffer (10 µl, pH 7.5) to prepare a mixed solution. A plasma sample (20 µl) prepared by a conventional method from animal blood which was sampled with the lapse of time after administration of the pharmaceutical agent was added to the above-mentioned mixed solution and the enzyme reaction was started at 30°C. The absorbance after 0 h and 1 h was measured using a microplate reader (trade name: Multiskan Bichromatic, manufactured by Labsystems Ltd.) at a wavelength of 405 nm and an increase (ΔOds) was determined. At the same time, an increase (ΔOdc) in absorbance of the reaction mixture containing the plasma prepared from the blood drawn before administration of the pharmaceutical agent, and an increase (ΔOdb) in absorbance of the reaction mixture without the plasma were determined and the relative enzyme activity of DPP-IV wherein DPP-IV activity before administration of the pharmaceutical agent was 100% was calculated from the following formula:

$$[(\Delta Ods-\Delta Odb)/(\Delta Odc-\Delta ODb)]\times 100$$

The results are shown in Table 3. In the Table, the DPP-IV activity value shows mean±standard deviation (n=3).

Table 3

| DPP-IV activity (%) | | |
|---|---|---|
| Time (h) | Tablet of Example 13 | Tablet of Example 13 + tablet of Reference Example 1 |
| 0 | 100±0 | 100±0 |
| 0.5 | 85±2 | 37±23 |
| 1 | 70±18 | 22±3 |
| 2 | 60±14 | 28±5 |
| 4 | 59±13 | 45±7 |
| 8 | 72±4 | 63±8 |

From Table 3, it has been clarified that the sustained-release preparation of the present invention obtained in Example 13 is capable of decreasing the plasma DPP-IV-inhibitory activity by 28 to 78% over the period of 1 hr to 8 hr after the administration by a single administration thereof or concurrent administration with the quick release preparation obtained in Reference Example 1.

**Experimental Example 4**

The effect of an organic acid on the dissolution behavior of the sustained-release preparation of the present invention was evaluated by a dissolution test.

To be specific, each tablet obtained in Examples 7 and 21 was subjected to a dissolution test (Paddle Method, The Japanese Pharmacopoeia Degradation Test 1st fluid (hereinafter to be abbreviated as The Japanese Pharmacopoeia 1st fluid) 500 mL, number of rotation 100 rpm). In addition, in the same manner as above except that the eluent was a CTAB-containing The Japanese Pharmacopoeia 2nd fluid, a dissolution test of the tablets obtained in Examples 7 and 21 was performed. The results are shown in Table 4 and Table 5, respectively.

Table 4

| Dissolution rate (%) | | |
|---|---|---|
| Time (h) | Example 7 | Example 21 |
| 0 | 0 | 0 |
| 1 | 35 | 40 |
| 2 | 52 | 58 |

Table 4 (continued)

| Dissolution rate (%) | | |
|---|---|---|
| Time (h) | Example 7 | Example 21 |
| 4 | 81 | 83 |
| 6 | 105 | 103 |

Table 5

| Dissolution rate (%) | | |
|---|---|---|
| Time (h) | Example 7 | Example 21 |
| 0 | 0 | 0 |
| 1 | 3 | 24 |
| 2 | 6 | 34 |
| 4 | 13 | 51 |
| 6 | 20 | 67 |
| 8 | 26 | 78 |
| 10 | 32 | 88 |
| 14 | 44 | 95 |
| 18 | 55 | 95 |

From Table 4 and Table 5, it has been clarified that the dissolution behavior (dissolution pattern in The Japanese Pharmacopoeia 1st fluid and CTAB-containing The Japanese Pharmacopoeia 2nd fluid) of the sustained-release preparation of the present invention can be controlled by the addition of an organic acid (citric acid).

**Industrial Applicability**

The sustained-release preparation, pharmaceutical agent and DPP-IV inhibitor-containing release control preparation of the present invention can appropriately inhibit DPP-IV activity for a long time. Therefore, the sustained-release preparation is useful as a pharmaceutical product (e.g., an agent for the prophylaxis or treatment of diabetes) superior in convenience and compliance, which is free of side effects and can be administered once a day.

## Claims

1. A sustained-release preparation comprising a dipeptidyl peptidase IV inhibitor and a hydrophilic polymer.

2. The sustained-release preparation of claim 1, wherein the content of the hydrophilic polymer in the preparation is not less than 5 wt%.

3. The sustained-release preparation of claim 1, which is used for the prophylaxis or treatment of diabetes.

4. The sustained-release preparation of claim 1, which is a hypoglycemic agent.

5. A pharmaceutical agent comprising two or more kinds of dipeptidyl peptidase IV inhibitor-containing preparations in combination, which have different release rates of dipeptidyl peptidase IV inhibitor.

6. The pharmaceutical agent of claim 5, which comprises a sustained-release preparation comprising a dipeptidyl peptidase IV inhibitor and a quick release preparation comprising a dipeptidyl peptidase IV inhibitor.

7. The pharmaceutical agent of claim 5, which is used for the prophylaxis or treatment of diabetes.

**8.** The pharmaceutical agent of claim 5, which is a hypoglycemic agent.

**9.** A dipeptidyl peptidase IV inhibitor-containing release control preparation capable of decreasing dipeptidyl peptidase IV activity in plasma by 10 to 90% at 1 hr after the administration.

**10.** A dipeptidyl peptidase IV inhibitor-containing release control preparation capable of decreasing dipeptidyl peptidase IV activity in plasma by 10 to 90% at 8 hr after the administration.

**11.** A dipeptidyl peptidase IV inhibitor-containing release control preparation capable of decreasing dipeptidyl peptidase IV activity in plasma by 10 to 90% over the period of from 1 hr to 8 hr after the administration.

**12.** The preparation of any of claims 9, 10 and 11, which is used for the prophylaxis or treatment of diabetes.

**13.** The preparation of any of claims 9, 10 and 11, which is a hypoglycemic agent.

**14.** A method of treating diabetes, which comprises administering the preparation of any of claims 9, 10 and 11 to a mammal.

**INTERNATIONAL SEARCH REPORT**

International application No. PCT/JP03/11570

A. CLASSIFICATION OF SUBJECT MATTER
Int.Cl[7] A61K45/00, 31/472, 38/55, 47/30, 47/38, 31/425, A61P3/10

According to International Patent Classification (IPC) or to both national classification and IPC

B. FIELDS SEARCHED

Minimum documentation searched (classification system followed by classification symbols)
Int.Cl[7] A61K45/00, 31/472, 38/55, 47/30, 47/38, 31/425, A61P3/10

Documentation searched other than minimum documentation to the extent that such documents are included in the fields searched

Electronic data base consulted during the international search (name of data base and, where practicable, search terms used)
MEDLINE/CAPLUS/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| Y | WO 02/062764 A1 (TAKEDA CHEMICAL INDUSTRIES LTD.), 15 August, 2002 (15.08.02), Claims 17, 18; examples 212, 162 & JP 2003-238566 A | 1-13 |
| X<br>Y | WO 01/72290 A2 (PROBIODRUG GESELLSHAFT FUR ARZNEIMITTELFORSCHUNG MBH.), 04 October, 2001 (04.10.01), Claims; page 6, lines 19 to 22; page 7, lines 12 to 14 & JP 2003-528135 A | 1-4,9-13<br>5-8 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>25 November, 2003 (25.11.03) | Date of mailing of the international search report<br>09 December, 2003 (09.12.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/11570

C (Continuation). DOCUMENTS CONSIDERED TO BE RELEVANT

| Category* | Citation of document, with indication, where appropriate, of the relevant passages | Relevant to claim No. |
|---|---|---|
| Y | Pospisilik JA et al., 'Long-term treatment with the dipeptidyl peptidase IV inhibitor P32/98 causes sustained improvements in glucose tolerance, insulin sensitivity, hyperinsulinemia, and beta-cell glucose responsiveness in VDF (fa/fa) Zucker rats.' Diabetes., 2002 April; 51(4): 943-50. | 1-13 |
| Y | WO 01/22941 A1 (LUNDBECK & CO AS H), 05 April, 2001 (05.04.01), Claims & JP 2003-510266 A | 1-13 |
| Y | WO 00/21525 A2 (NOVARTIS ERFIND VERWALT GMBH.), 20 April, 2000 (20.04.00), Claims & JP 2002-527388 A | 1-13 |
| Y | WO 99/58114 A1 (Freund Industrial Co., Ltd.), 18 November, 1999 (18.11.99), Claims & JP 2000-034224 A | 1-13 |
| Y | EP 284849 A1 (LEK TOVARNA FARMACEVTSKIH), 05 October, 1988 (05.10.88), Claims & JP 63-290818 A | 1-13 |
| Y | WO 01/047557 A1 (AJINOMOTO KABUSHIKI KAISHA), 05 July, 2001 (05.07.01), Claim 3 & EP 1258249 A | 5-13 |
| Y | JP 2002-179554 A (Takeda Chemical Industries, Ltd.), 26 June, 2002 (26.06.02), Claim 1 (Family: none) | 5-13 |
| Y | EP 1110541 A1 (JOHNSON & JOHNSON CONSUMER), 27 June, 2001 (27.06.01), Claim 1 & JP 2001-278779 A | 5-13 |
| Y | Mace KF et al., 'The pharmacokinetics of LY307161 SR, a sustained release formulation of a DPP-IV resistant GLP-1 analog, administered by subcu taneous injection in rats and dogs. 'Diabetol ogia, (August, 2002), Volume 45, Number Supplement 2, pp.A176 | 1-13 |

Form PCT/ISA/210 (continuation of second sheet) (July 1998)

**INTERNATIONAL SEARCH REPORT**

International application No.
PCT/JP03/11570

C (Continuation). DOCUMENTS CONSIDERED TO BE RELEVANT

| Category* | Citation of document, with indication, where appropriate, of the relevant passages | Relevant to claim No. |
|---|---|---|
| Y | Byrd RA et al., 'Reproductive and developmental toxicity studies in rats and rabbits with LY307161 SR, a sustained-release formulation of a dipeptidyl peptidase-IV (DPP-IV), resistant glucagon-like peptide 1(GLP-1), analog. 'Teratology, (June, 2002), Vol. 65, No. 6, pp.339 | 1-13 |

Form PCT/ISA/210 (continuation of second sheet) (July 1998)

**INTERNATIONAL SEARCH REPORT**

International application No.
PCT/JP03/11570

**Box I Observations where certain claims were found unsearchable (Continuation of item 2 of first sheet)**

This international search report has not been established in respect of certain claims under Article 17(2)(a) for the following reasons:

1. [X] Claims Nos.: 14
because they relate to subject matter not required to be searched by this Authority, namely:
Claim 14 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [X] Claims Nos.: 1-13
because they relate to parts of the international application that do not comply with the prescribed requirements to such an extent that no meaningful international search can be carried out, specifically:
(See extra sheet.)

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP03/11570

Claims 1 to 13 relate to sustained release preparations (claims 1 to 4)/drugs (claims 5 to 8)/release controllers (claims 9 to 13) each containing as the active ingredient a compound defined by the desired property "dipeptidyl peptidase IV inhibitor". Although claims 1 to 13 involve any compounds having this property, only part of the claimed compounds are disclosed in the meaning within PCT Article 5. Thus, it is recognized that these claims are not supported by the disclosure of the description in the meaning within PCT Article 6.

Even though the common technical knowledge is taken into consideration, the scope of the compound serving as "dipeptidyl peptidase IV inhibitor" cannot be specified. Thus, claims 1 to 13 do not comply with the clearness in accordance with PCT Article 6 too.

Such being the case, the search was made on the relationship between a dipeptidyl peptidase IV inhibitor and a sustained release preparation and sustained release preparations/drugs/release controllers containing, as the active ingredient, the compounds which are specifically illustrated in the description and the sustained release properties of which were discussed, i.e., the compounds A and B.

Form PCT/ISA/210 (extra sheet) (July 1998)